# EUROPEAN PATENT APPLICATION

(11) **EP 2 602 245 A1**
(43) Date of publication of application: **12.06.2013**
(21) Application number: 11192011.2
(22) Date of filing: 05.12.2011
(51) Int. Cl.: C07C 273/04

(54) **A process for synthesis of urea and a related arrangement for a reaction section of a urea plant**

(71) Applicant: Urea Casale SA, 6900 Lugano-Besso (CH)
(72) Inventor: Sioli, Giancarlo, 22010 Cernobbio (CO) (IT); Cavuoti, Giacomo, 6900 Lugano (CH)
(74) Representative: Zardi, Marco

(57) **Abstract**

A process for synthesis of urea and a related reaction section of a urea plant, where: ammonia and carbon dioxide are reacted in a liquid phase in a first reaction zone (S1) and heat (Q1) is withdrawn from said first reaction zone to promote the formation of ammonium carbamate, the liquid product (103) from said first reaction zone is then passed to a second reaction zone (S2) distinguished from said first reaction zone, and heat (Q2) is added to said second reaction zone to promote the decomposition of ammonium carbamate into urea and water, where the liquid phase in at least one of said first reaction zone and second reaction zone is kept in a stirred condition.

## Description

### DESCRIPTION

### Field of the invention

The invention relates to conversion of ammonia and carbon dioxide into urea. The invention relates more in detail to a novel process and arrangement for the reaction section of a urea plant.

### Prior Art

Urea is formed by reaction of ammonia with carbon dioxide, according to consecutive equilibrium reactions:

2 NH₃ + CO₂ ↔ NH₄⁺ + NH₂-COO ⁻ ↔ NH₂-CO-NH₂ (urea) + H₂O

Formation of urea then involves a fast and strongly exothermic reaction between ammonia and carbon dioxide bringing to ammonium carbamate, and a slower, slightly endothermic reaction of ammonium carbamate forming urea and water. The second and slower reaction constitutes the rate-determining step of the overall chemical synthesis.

Early processes for synthesis of urea were operated at about 400 bar, with a reactor structured as a simple vertical cylindrical pressure vessel. These processes were able to achieve a good CO₂ conversion to urea (up to 80%), but suffered a low efficiency in the recovery of non-reacted NH₃ and CO₂ and practical drawbacks due to the very high pressure. Introduction of total recovery of non-converted chemicals allowed accepting lower CO₂ conversion rates (64-70%), while reducing the operating pressure down to 200-250 bar. Reducing the pressure has, of course, significant advantages in terms of the cost of the pressure vessels and other equipments, as well as energy demand for pumps and compressors.

In the known art, the above sequence of reactions is carried out by feeding NH₃ and CO₂ at the bottom section of a vertical reactor usually having a large height to diameter ratio. The largely exothermal reaction between the NH£ and CO2 feed and formation of ammonium carbamate takes place substantially in the lower section of the reactor, while the endothermal, slower formation of urea takes place in an upper part of the reactor. The reaction products are then crossed by an up-flow of co-current, reacting gas and liquid phases.

The conversion rate which is reached inside the reactor is substantially conditioned by mass transfer rates, in competition with rates of the chemical reactions. A urea synthesis reactor is at least partially a vapour-liquid heterogeneous reaction system where: the vapour phase contains free CO₂, NH₃, some water and inert gases; the liquid phase mainly contains NH₃, ammonium carbamate, urea, water and some ammonium carbonate. The reactants are progressively transferred from the vapour to the liquid phase, wherein CO₂ reacts with NH₃ to form the ammonium carbamate, and successively urea and water. As a consequence of the diffusion rates, chemical equilibria tend to establish, at the vapour-liquid interface, between CO₂, NH₃, H₂O in the gaseous phase and, respectively, dissolved in the liquid phase.

Attempts to improve the conversion rate have focused inter alia on the design of the reactor. For example, the provision of internal perforated plates, dividing the reactor into compartments, yielded significant conversion improvements. In this respect, US 5,304,353 discloses a reactor operating with the insertion of contact plates; US 5,750,080 discloses a method for in-situ modernisation of a reactor provided with internal, perforated plates, consisting in the addition of structurally independent caps, achieving a better gas-liquid intermixing situation; US 6,120,740 discloses reactor plates where perforations are arranged in a way to better control the liquid flow, increasing the reactor yield with the result of reducing the need to recycle non-reacted products. The latest reactors, provided with specially designed internal plates, operate the reaction at 140-160 bar, with CO₂ conversion in the range 58-62%.

Hence, it can be stated that for a given set of parameters including temperature, pressure, residence time, NH₃/CO₂ mole ratio and H₂O/CO₂ mole ratio, the efficiency of the reactor is also strongly influenced by the reactor internal design. For example, the installation of internal perforated plates is improving the gas-liquid contact, and hampering, at least partially, the internal back-mixing of products with reactants.

However, there are some drawbacks which have not yet been completely solved, including the dangerous back-mixing of products with reactants which affects the conversion rate; moreover there is an ongoing incentive and a substantial interest to seek a design capable of further raising the conversion rate to urea without increasing, or even decreasing, the pressure level.

### Summary of the Invention

The problem underlying the present invention is to improve the efficiency of the known process for urea production by acting on the configuration of the reactor or reaction section of a urea plant.

The above problem is solved with a process for synthesis of urea from reaction of ammonia and carbon dioxide, characterized in that:
- ammonia and carbon dioxide are reacted in a liquid phase and in a first reaction zone, and heat is withdrawn from said first reaction zone to promote the formation of ammonium carbamate, said first reaction zone producing a first liquid product mainly comprising ammonium carbamate, ammonia and water;
- said first product is then passed to a second reaction zone distinguished from said first reaction zone, and heat is added to said second reaction zone to promote the decomposition of ammonium carbamate into urea and water, said second reaction zone producing a second liquid product containing urea, residual unconverted carbamate and excess ammonia, and
- the liquid phase in at least one of said first reaction zone and second reaction zone being kept in a stirred condition.

The term of second reaction zone distinguished from said first reaction zone shall be understood in the sense that a reaction section of a urea plant for the above process includes a well recognizable zone (the first reaction zone) dedicated to the formation of ammonium carbamate, and a well recognizable zone (the second zone) dedicated to formation of urea. The first zone and second zone may be separated by a physical boundary, although this is not mandatory. In some embodiments the first zone and second zone are in different pressure vessels, e.g. in a first and second vessel, thus being physically separated. In some other embodiments the first zone and second zone may be arranged inside the same vessel, e.g. being an upper part and a lower part of an elongated vertical vessel.

The stirred condition shall be understood as a mechanical agitation, which may be induced for example by rotary means. Suitable means include turbines, impellers or the like. In a preferred embodiment, said stirred condition for the first and/or the second reaction zone is provided in a fully-baffled condition of the liquid phase. Definition of the fully-baffled condition will be given below.

The invention discloses to carry out the conversion of ammonia and carbon dioxide into urea with a step-wise advancement in a series of reaction zones. The invention provides a separation between two reaction zones where the fast, exothermic formation of carbamate and, respectively, the slower endothermic dissociation into urea and water are promoted. By withdrawing heat from the first reaction zone, the formation of carbamate is promoted whilst the endothermic formation of urea is not favoured, hence a first product is substantially a solution of ammonium carbamate, ammonia and water; vice-versa, the formation of urea is promoted in the second reaction zone by adding heat. The stirred condition also plays an important role especially for enhancing the heat transfer to the liquid phase, and therefore promoting the reaction rate.

In a preferred embodiment, said second reaction zone has a temperature higher than the temperature of said first reaction zone. More preferably said second reaction zone has substantially the same pressure of said first reaction zone. More preferably the second reaction zone has a temperature higher than the first one, and substantially the same pressure. For example the first reaction zone is run at around 150 °C and the second reaction zone is run at around 180 °C. Preferred ranges are 150-160 °C for the first zone and 170-190 °C for the second zone.

In a preferred embodiment, the process involves also a third reaction zone, which can also termed a stripping zone, fed with a flow of second liquid product obtained in the second zone, and where the residual carbamate contained in said second liquid product is decomposed by means of a heat supply and optionally by means of addition of a stripping medium, releasing ammonia and carbon dioxide. More preferably the liquid phase in said third reaction zone is also kept in a stirred condition and preferably a strongly stirred condition.

More in detail, a gaseous stream containing NH₃ and CO₂ from decomposition of carbamate, plus some NH₃ excess, is obtained in said third zone, and is sent back to the first reaction zone for recovery purposes. The stripping of residual carbamate can be promoted in the stripping zone by adding heat and/or by adding a stripping medium such as carbon dioxide. Said stripping zone delivers a concentrated urea solution which is transferred to a downstream urea separation process, for removing water and possibly for recovering further amounts of ammonia and carbon dioxide from low pressure carbamate solution, according to a known technique.

Preferably, a gaseous stream comprising at least part of said ammonia and carbon dioxide, released in the third reaction zone by means of the stripping process, is fed directly in the gaseous state into said first reaction zone. This is a considerable advantage because a high-pressure condenser is no longer necessary, contrary to the prior art of urea processes like e.g. the conventional self-stripping or CO2-stripping processes, where a high-pressure condenser is deemed necessary.

Thanks to the fast, somewhat turbulent motion of the stirred liquid phase in the first reaction zone, the gaseous ammonia and CO2 entering the first zone will be brought to intimate contact with the liquid phase, recovering them by reaction to ammonium carbamate. Hence, previous condensation is no longer necessary, although it is possible in some embodiments.

Preferably, the gaseous flow of ammonia and carbon dioxide from the third zone is directed close to the stirring means operating in the first zone, for example close to the rotating blades of an impeller, to enhance the above effect.

According to several embodiments of the invention, any of the first reaction zone and second reaction zone may be arranged in a single vessel or more vessels or groups of vessels. Said embodiments could be mixed e.g. using one vessel for the first reaction zone, and a plurality of vessels for the second reaction zone. Physical separation between said reaction zones can be obtained with a partition wall, when the reaction zones are contained in the same vessel, although a separation wall is not a necessary feature.

If the first reaction zone and second reaction zone are comprised in the same vessel, preferably the first reaction zone is above the second reaction zone. In a preferred arrangement of a single-vessel embodiment, a pressure vessel has an upper zone forming the first reaction zone, a central zone forming the second reaction zone, and a bottom part forming the stripping zone.

Also the stripping zone may be included in the same, single vessel containing the first and second reaction zones as in the above example, or may be realized with a dedicated vessel or vessels. Preferably the stripping zone has a single, dedicated vessel. In preferred embodiments, the carbamate solution (liquid product from the second zone) and the stripping medium, if any, are directed close to rotating blades of an impeller or turbine working in the third zone, so to promote the stripping effect. The impeller may be surrounded by a heating coil which provides the necessary heat for the stripping process.

The stirred condition, as stated above, is preferably in accordance with the so-called fully-baffled condition of the liquid phase. A fully-baffled condition is known to a skilled person and a definition can be found in literature; to summarize, it is defined as a condition where the tangential entrainment of liquid is impeded, for example by appropriate baffles, and the cylindrically rotating vortex disappears, allowing transfer of a significant deal of power to the liquid under agitation.

Mechanical agitation is provided for example with one or more impellers. As a rough indication, the power transferred from the impellers to the liquid phase is preferably 0.2 to 2 kW per cubic meter of un-gassed liquid, more preferably 0.4 to 1.5 kW per m³. Hence impellers are preferably designed to deliver such power to the liquid phase, when they are in use.

Excess, humid gases from the total process are discharged from the first reaction zone and throttled to control the pressure of the system.

The steps of withdrawing or adding heat are performed with heat exchange means such as, for example, a coil traversed by a cooling medium or, respectively, a heating medium. The heat exchange means are preferably immersed in the liquid phase.

The above process has been found surprisingly efficient in solving the problems left by the known art. An advantage of the invention is the achievement of good momentum transfer conditions, thus favouring the progress of the chemical reactions involved. In addition, by means of a separation between the first and the second reaction zone, possibly in separate vessels or separate chambers of a vessel, the invention can reduce in a substantial manner the dangerous back-mixing of products with reactants. A cascade of reactors, in particular, is able to avoid said back mixing.

A further advantage of the invention is that the first and second zone can be designed according to specific needs. For example, a single, stirred tank reactor may suffice for providing the first reaction zone dedicated to the fast exothermal reaction between NH₃ and CO₂; although one or more successive vessels may accomplish to the duty of the second zone, dedicated to the relatively slow, endothermal formation of urea. Finally a single, stirred tank vessel, may be individuated as the third zone, taking care of the gas stripping operation.

The heat exchange at process side, which is usually limiting the overall heat removal or supply to the reacting mass, is substantially enhanced by a mechanically stirred reactor configuration, reducing the extension of the heat exchange surface, and the reactor volume, in comparison to reactors of the known art, at equality of urea production rate per unit time.

The conversion degree of the carbon compound, without changing the operation temperature with respect to the know art, is also markedly increased.

An object of the invention is also a reaction section of a plant for synthesis of urea from ammonia and carbon dioxide, for carrying out the above process. In a general embodiment, the reaction section includes:
- a first reaction zone for conversion of ammonia and carbon dioxide into ammonium carbamate and a second reaction zone for decomposition of carbamate into urea, said second reaction zone being distinguished from said first reaction zone;
- means for feeding ammonia and carbon dioxide to said first reaction zone, and cooling means disposed in the first reaction zone and adapted to remove the heat of formation of ammonium carbamate,
- means for feeding a first product, mainly comprising ammonium carbamate, ammonia and water, from said first reaction zone to said second reaction zone;
- heating means disposed in the said second reaction zone, adapted to provide heat for the decomposition of part of said carbamate into urea, and a flow line for removing a second product containing urea, residual unconverted carbamate and excess ammonia from said second reaction zone, and
- stirring means arranged in at least one of said first reaction zone and second reaction zone and preferably in both said first and second reaction zone.

Preferably the reaction section includes a third reaction zone, or stripping zone; means feeding a flow of said second liquid product from the second zone to said third zone; heating means and optionally a line for addition of a stripping medium to said third zone; stirring means for keeping the liquid phase in said third reaction zone in a stirred condition. More preferably there is provided a gas flow line for a direct connection between said third zone and first zone, arranged to recycle a gaseous flow comprising ammonia and carbon dioxide, which is released in the third reaction zone, into said first reaction zone. Even more preferably, said flow line is arranged to direct said gaseous flow close to stirring means which operates in the first reaction zone.

The reaction zones can be obtained with one or more vessels, or multi-compartmented vessels. Some of the possible embodiments will be described below as examples. As apparent to the skilled person, other equivalent embodiments are possible, with multiple vessels, compartmented vessels or any combination thereof.

### First embodiment

The equipment comprises a single, vertically elongated vessel, comprising all the above three zones, and crossed by the liquid stream downwards, which means in the opposite direction, with reference to the known art.

The upper end of the vessel constitutes the first zone, wherein the reaction of ammonia and carbon dioxide is taking place, preferably under strong agitation, and heat is removed by a cooling coil internally crossed by a cooling fluid.

The mid part of the vessel is the second zone, where ammonium carbamate is left to decompose into urea and water. Heat may be supplied through a coil, to accelerate the conversion rate.

The lower end of the vessel is performing, preferably under strong agitation and increased temperature, the residual carbamate decomposition and the NH₃ excess stripping. This operation may also be favoured by additional injection of CO₂ as stripping medium. Heat is preferably supplied by a heating coil, crossed by a heating fluid. The resulting gas stream may be carried up to reach the top zone, wherein it may be recovered into the carbamate formation.

### Second embodiment

The reaction zones comprise two separate stirred-tank reactors arranged in cascade, namely a first reactor providing the first reaction zone, and a second reactor providing the second reaction zone.

Each reactor is preferably equipped with a mechanical agitator; the first vessel is also equipped with a cooling coil, internally crossed by a cooling fluid, while the second vessel is equipped with a heating coil crossed by a heating fluid. In operation, NH₃ and CO₂ are fed to the first reactor, wherefrom out-flowing fluids are passed to the second reactor, wherefrom the urea solution is passing to a third vessel, where the residual carbamate is decomposed, and the resulting CO₂, if desired with additional fresh CO₂, is intimately contacted with the liquid phase by means of an adequate stirrer, with the aim of stripping out the unreacted ammonia excess, which is recycled back to the first reaction vessel.

### Third embodiment

The equipment comprises a cascade of stirred tank type, vertical reactors, each constituting a separate vessel. A single reactor, for example, provides the first reaction zone, while three further vertical reactors form the second reaction zone. Each reactor is equipped with an internal, mechanical agitator, and a heat exchanger, removing or supplying heat respectively in reactors of the first or second zone. Ammonia and CO₂ are fed to the first reactor, and the fluids overflow from that reactor to the first reactor of the second stage. The last reactor of the second series delivers the final product to the final decomposition and stripping unit, wherefrom the gaseous phase is recycled back to the initial reactor of the total series.

### Fourth embodiment

The second reaction zone is realised by means of a horizontal reactor, providing a series of internal compartments for the second reaction zone. Said compartments are separated by internal weirs, overflowing the liquid phases from each compartment to the next one. Each compartment is equipped with a mechanical agitator; coolers are heaters are accommodated in the various compartments, following the already described criteria.

The invention is now described in further details with reference to preferred while non-limiting embodiments, and with the help of the drawings.

### Brief Description of the Drawings

Fig. 1 is a block scheme of a process according to a preferred embodiment of the invention.
Fig.2 is a scheme of an equipment for carrying out the process, in accordance with a single-vessel embodiment.
Fig 3 is a scheme of an equipment according to a multiple-vessel embodiment, including two stirred-tank reactors and a stripper.
Fig. 4 is a scheme of an embodiment including a cascade of stirred-tank reactors, and a stripper.
Fig. 5 is a scheme of an embodiment alternative to Fig. 4, wherein the cascade of reactors for the second reaction zone is replaced by a horizontal reactor, provided with internal, mechanically stirred compartments.

### Detailed Description of preferred embodiments of the invention

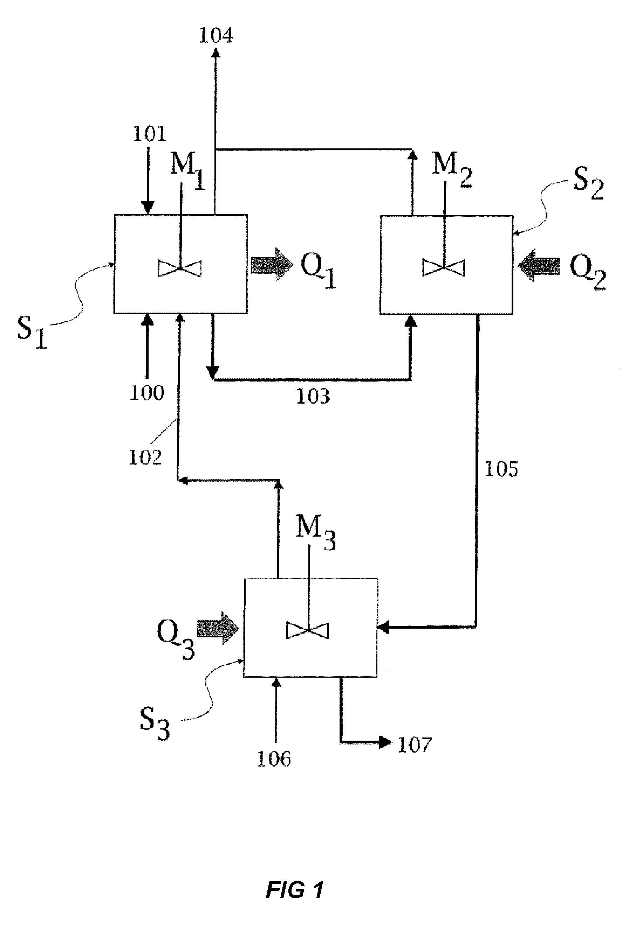

Referring to the block scheme of Fig. 1, the high-pressure conversion of carbon dioxide and ammonia into urea is carried out with a first step in a first reaction zone S1, followed by a second step in a second reaction zone S2.

A gaseous stream 100 of carbon dioxide and a liquid stream 101 containing ammonia make-up and some carbamate recycle are added to said reaction zone S1, where a liquid phase is maintained in agitation by a suitable mixer M1. A strong heat flow is released by the fast, exothermal conversion of ammonia and carbon dioxide into ammonium carbamate, and heat Q1 is removed from said reaction zone S1 to maintain the desired reaction temperature for the formation of ammonium carbamate. Heat Q1 is removed by appropriate means, e.g. by a heat exchanger crossed by a cooling medium.

The liquid phase is taken from reaction zone S1 and passed to the subsequent reaction zone S2 via line 103. The temperature of the liquid phase in the reaction zone S2 is similar or preferably higher than temperature of the liquid phase in zone S1, thus favouring the endothermic decomposition of ammonium carbamate into urea and water. This is achieved by supplying heat Q2 to zone S2 by appropriate means, e.g. a heat exchanger crossed by a heating medium.

The pressure in the second zone S2 may be substantially the same as in the first zone S1. Preferably said pressure is in the range 120 to 160 bar. The liquid phase in said second zone S2 is kept in agitation by a suitable mixer M2, enhancing the transfer of heat Q2 to the liquid mass.

A concentrated aqueous solution of urea, with residual non-converted carbamate, is obtained at line 105, while a gaseous phase, mainly consisting of ammonia, carbon dioxide, water vapour and inert gases, is vented out from zones S1 and S2 via the line 104. Said line 104 may be throttled for the purpose of pressure control of the whole system.

A third reaction zone, or stripping zone, S3 is dedicated to the removal of unconverted carbamate and excess NH₃ from the reaction product 105 (urea solution), via thermal decomposition and gas stripping process. Optional addition of a stripping medium such an inert gas stream, or carbon dioxide, is indicated by line 106. The gaseous products leave said third zone S3 through the line 102, and are redirected to the first reaction zone S1, where they are partially recovered as reactants. Heat is supplied to zone S3 by appropriate means, e.g. a heat exchanger crossed by a heating medium, preferably reaching temperatures exceeding 200°C. A more concentrated urea aqueous solution is delivered by line 107. In some embodiments the redirection of gaseous products from the third zone to the first zone may require a gas compressor or a blower (not shown in the figures).

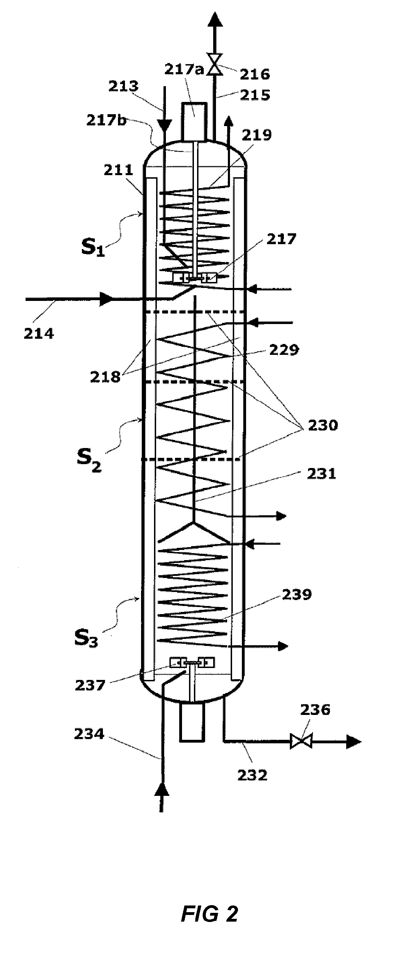

Each of the zones S1, S2 or S3 can be implemented with one or more reactor vessels. In particular, the zones S1 and S2 may be implemented with a cascade of reactors or partitioned reactors. Some preferred embodiments of the outlined technology are presented below, with reference to Fig. 2, 3 and 4.

### First embodiment

Referring to Fig. 2, reaction zones S1 and S2 are respectively the upper and the mid part of a vessel 211. Preferably, as shown, said vessel 211 is an elongated, vertical vessel.

Said vessel 211 includes a top mixing turbine 217 and an upper heat exchange coil 219; another heat exchange coil 229 in the mid-part; perforated trays 230 and a line 231 for recovery of gaseous reactants; a bottom mixing turbine 237 and a bottom heat exchange coil 239. Baffles 218 are extended to the whole height of the vessel 211 to realize a "fully baffled" condition as explained above. The impeller 217 has a driving motor 217a and a shaft 217b extending inside the vessel 211. The mixer is preferably a magnetically-driven machine, eliminating the problem of sealing the driving shaft.

It may be noted that, in order to exploit efficiently the heat transfer conditions, in connection to the mechanical agitation, the coil assembly 219 must not prevent the liquid circulation imparted by the mixing turbine 217. Some expedients can be adopted to this purpose, as for instance by keeping the coil bank sufficiently away from the shell of the vessel 211, and by keeping a reasonable clearance between successive coils.

Ammonia, usually with some recycle of carbamate solution, is introduced via a liquid duct 213 at the top of vessel 211, in proximity of the upper face of the mixing turbine 217. Carbon dioxide is added via line 214 to the liquid phase in the vessel 211, preferably in proximity of the mixing turbine.

The product of reaction, mainly comprising carbamate, ammonia and water, flows downwards to cross the reaction zone S2. The liquid volume in S2 may be significantly larger than the volume of the first reaction zone S1, due to the relatively lower reaction rate. The heat supply from the coil 229 controls the temperature of the vessel content. The S2 zone is preferably equipped with the perforated plates 230, as used in the state of art technologies.

Finally, the liquid phase reaches the lowest part of the vessel 211 where, at higher temperature, CO₂ is evolved, and possibly added through the line 234, in proximity of the lower face of the mixer 237, with the aim of stripping out the residual excess of dissolved ammonia. The resulting gaseous stream, comprising water-saturated CO₂ and NH₃, flows up in the direction of the mixer 217, carried by the line 231, to be recovered in the upper first reaction zone S1.

The urea aqueous solution constituting the final product is available at line 232. The outflow is controlled by the valve 236, actuated on the basis of the liquid level inside the vessel. A residual gas stream is discharged from top of reactor 211 through a line 215, where a manual or automatic valve 216 controls the pressure inside the reactor itself.

### Second embodiment

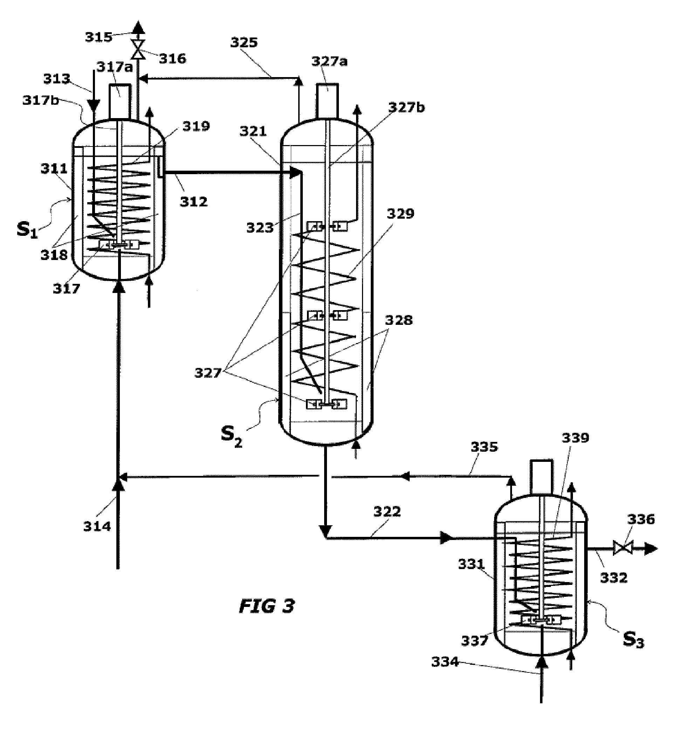

Referring to Fig. 3, reaction zones S1 and S2 are now obtained with a first stirred vessel 311 and a second stirred vessel 321, connected by a transfer line 312. A third stirred vessel 331 provides the stripping zone S3.

Vessels 311, 321 and 331 have a similar structure. They are equipped with respective mixing turbines 317, 327 and 337. References 317a, 317b, 327a, 327b denote motors and shafts. Preferably the turbines are magnetically-driven. Full-length vertical baffles 318, 328 are to realize a "fully baffled" condition

The liquid volume in S2 may be significantly larger than the volume of the first reaction zone S1, due to the relatively lower reaction rate. Due to larger volume of liquid, the second vessel 321 is usually larger than the other ones, in particular than the first vessel 311. The turbine 327 may comprise several blade sections mounted on a shaft 327b, to keep uniform agitation in said vessel 321.

The vessels also contains respective heat exchangers. In particular, a coil 319 is arranged to remove heat from the first reaction zone S1 in vessel 311, while the coils 329 and 339 supply heat to the zones S2 and S3.

Ammonia, usually with some recycle of carbamate solution, is introduced via the liquid duct 313 into the agitated vessel 311, in proximity of the upper face of said mixing turbine 317. Carbon dioxide is added via line 314 to the liquid phase in the vessel 311, in proximity of the lower face of the mixing turbine. A residual gas stream is discharged by reactor 311 through the line 315, where the manual or automatic valve 316 controls the pressure inside the reactor itself.

The reactor product, mainly comprising carbamate, ammonia and water, is collected by line 312, and transferred to the second stirred vessel 321, wherein it is released by the pipe 323 in proximity of the upper face of a mixer 327. A coil 329, located inside the vessel 321, is devised to supply heat, controlling the temperature of the vessel content.

The reactor 321 is vented together with the reactor 311 through the line 325, joining the line 315 upstream the valve 316.

The final liquid product, mainly urea in aqueous solution, is obtained at line 322 and is transferred to vessel 331. The required stripping action, necessary for the recovery of the surplus ammonia, is granted by the carbon dioxide resulting from the unconverted carbamate decomposition, with optionally added extra carbon dioxide, injected by a pipe 334 below the mixer 337. The resulting gaseous stream, comprising water-saturated CO₂ and NH₃, is transferred by the line 335, to be recovered inside the first reaction zone S1.

The urea aqueous solution, constituting the final product, is available at line 332. The outflow is controlled by the valve 336, actuated on the basis of the liquid level inside the vessel 331.

### Third embodiment

In this embodiment, the second reaction zone is set up with multiple stirred reactors, arranged in cascade or in series. The advantage is that back-mixing phenomena are minimised, in comparison to the previous embodiments, increasing the achievable conversion rate.

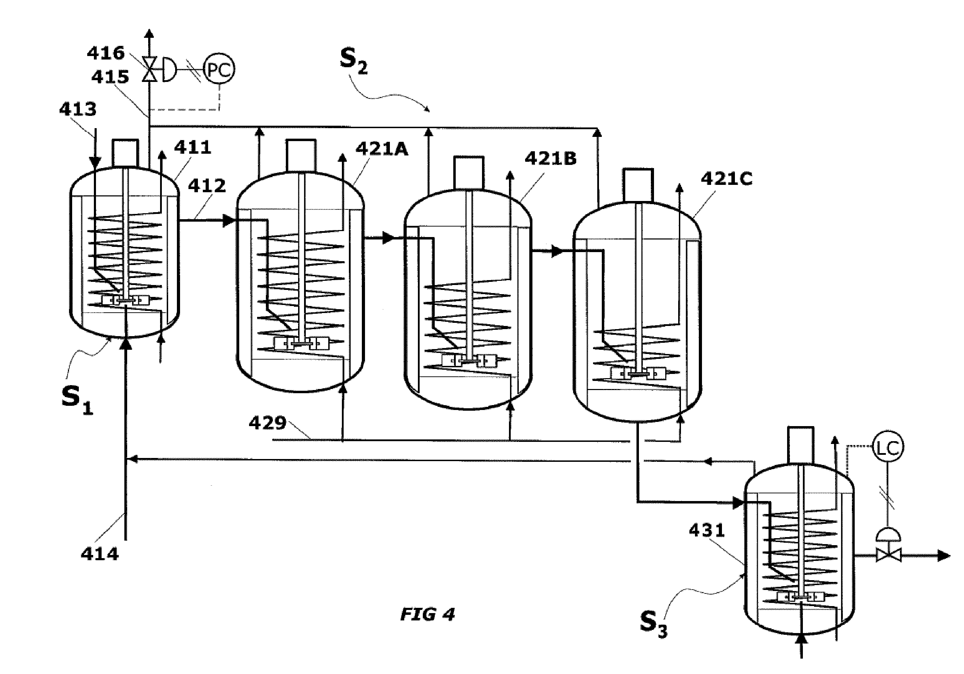

Referring to Fig. 4, the first reaction zone S1 is formed by vessel 411, while the second reaction zone S2 is formed by three vessels in cascade, items 421A, 421 B and 421C. The third zone or stripping zone is in a further vessel 431. Said vessels include mixing turbines and heat exchangers similarly to embodiments of Figs. 1 to 3.

The liquid ammonia feed enters the first vessel 411 via the pipe 413, while CO₂ is fed below the mixer of the same vessel via the pipe 414. The reaction heat removal is provided by banks of coils located inside the vessel, crossed by an adequate cooling fluid. The off gas is discharged by the pipe 415, and is used to control the system pressure by means of the valve 416.

The liquid product from the first vessel 411 is transferred by pipe 412 to the reactor 421A, namely the first reactor of the cascade, and carried in proximity of the mixer thereto, as indicated by the end of flow line 412, to be evenly distributed inside the vessel.

The liquid phase, which main component is ammonium carbamate, crosses in series the cascade of stirred vessels 421A, 421B and 421C, where the decomposition of the carbamate gives out progressively urea and water. A heating medium is supplied by the pipe 429 to the coil banks of the reactors, to compensate for the required endothermic heat. The final product, aqueous urea solution with excess ammonia, is discharged from the last reactor of the cascade, say 421C, to the next stripping vessel 431. Vent lines from the cascade join the line 415, as shown.

The vessel 431 has the same duty and operating conditions as 331 in Fig.3.

### Fourth embodiment

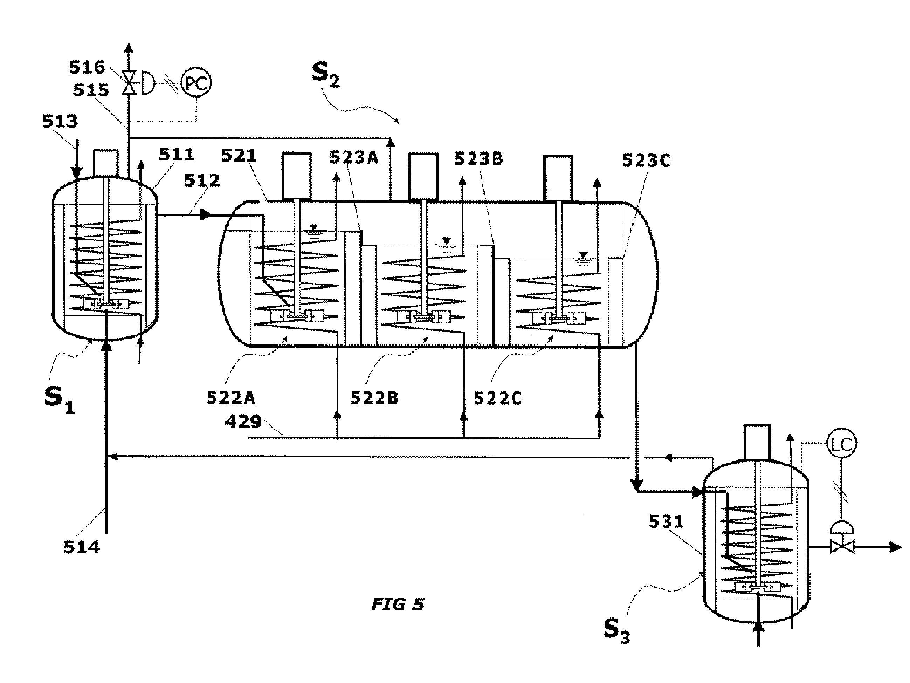

In this further embodiment, depicted in Fig.5, the second reaction zone S2 is realised by a single, multi-compartmented, horizontal vessel. A cylindrical, horizontal vessel 521 is partitioned in consecutive chambers or compartments as 522A, 522B and 522C, separated by frames 523A,523B and 523C, allowing the liquid phase to overflow from each chamber to the next one.

The first reactor vessel 511 is similar to reactors 311, 411 of the previously described embodiments. Each of the compartments in the vessel 521 has a mixing turbine and a heat exchanger.

The liquid phase from the first reactor 511, coming from line 512, crosses in series the three compartments inside the vessel 521, where urea and water are progressively obtained from the decomposition of the carbamate. A heating medium is supplied by the pipe 529 to the coil banks in the compartments 522A, 522B and 522C, to compensate for the required endothermic heat. The final product, aqueous urea solution, is discharged from the last compartment to the stripping vessel 531, as in the preceding embodiments.

### EXAMPLE

In a commercial unit, taken as a reference, producing 1000 MTPD (metric tons per day) of urea, NH₃ and CO₂ feeds, together with a carbamate recycle stream containing water, are fed into the bottom section of cylindrical, vertical reactor of 75 m³ internal volume, provided with specially perforated trays. The operating pressure is 160 bar, measured at reactor bottom section, where ammonia and recycle carbamate solution, plus gaseous CO₂, are introduced.

Under steady state conditions the reactor effluent leaves the reactor in the top section at 188°C. Said effluent is analysed in this example. The reactor material balance, based on reactor feeds, carbamate recycle solution analysis, and net urea produced, is checked as follows:

| | |
|---|---|
| urea formed in reactor | 34.2 % |
| CO₂ as unreacted carbamate | 14.7 % |
| free NH₃ plus NH₃ in carbamate | 31.3 % |
| total water | 19.8 % |

wherefrom:

| | |
|---|---|
| total CO₂ in reactor | 39.8 % |
| total NH₃ in reactor | 50.7 % |
| net water fed to reactor | 9.5 % |

and therefore:

| | |
|---|---|
| NH₃/CO₂ molar ratio | 3.30 |
| H₂O/CO₂ molar ratio | 0.58 |
| Conversion rate | 63 % |

In comparison to this commercial set up, a pilot reactor system according to a single-vessel embodiment, similar to Fig. 2 has been operated at 150 bar and 170 °C in the first reaction zone S1.

In this zone S1, heat is removed to maintain the above temperature, by circulation of pressurised water, generating low pressure steam in a separate drum. The liquid phase containing the carbamate is proceeding downwards to the zone S2, where urea is formed in practically isothermal conditions, and finally to the lower reactor end (zone S3), wherein the residual carbamate is decomposed at higher temperature (>200°C). The released CO₂ is stripping out some ammonia excess, this gaseous phase travelling upwards to the zone S1.

| | | |
|---|---|---|
| The resulting mass balance is as follows: | | |
| | urea formed in reactor | 43.5 % |
| | CO₂ as unreacted carbamate | 6.7 % |
| | free NH₃ plus NH₃ in carbamate | 24.8 % |
| | total water | 27.0 % |
| wherefrom: | | |
| | total CO₂ in reactor | 38.6 % |
| | total NH₃ in reactor | 49.5 % |
| | net water fed to reactor | 13.9 % |
| therefore: | | |
| | NH₃/CO₂ molar ratio | 3.32 |
| | H₂O/CO₂ molar ratio | 0.88 |
| | Conversion rate | 82.6 % |

## Claims

1. A process for synthesis of urea from reaction of ammonia and carbon dioxide, **characterized in that**:
- ammonia and carbon dioxide are reacted in a liquid phase and in a first reaction zone (S1), and heat (Q1) is withdrawn from said first reaction zone to promote the formation of ammonium carbamate, said first reaction zone producing a first liquid product (103) mainly comprising ammonium carbamate, ammonia and water;
- said first product is then passed to a second reaction zone (S2) distinguished from said first reaction zone, and heat (Q2) is added to said second reaction zone to promote the decomposition of ammonium carbamate into urea and water, said second reaction zone producing a second liquid product (105) containing urea, residual unconverted carbamate and excess ammonia, and
- the liquid phase in at least one of said first reaction zone and second reaction zone being kept in a stirred condition.

2. A process according to claim 1 or 2, said stirred condition being provided in a fully-baffled condition of the liquid phase.

3. A process according to claim 1, said second reaction zone having a temperature higher than temperature of said first reaction zone, and preferably said second reaction zone having substantially the same pressure of said first reaction zone.

4. A process according to any of claims 1 to 3, said first reaction zone and said second reaction zone being physically separated.

5. A process according to any of the preceding claims, said first reaction zone and said second reaction zone being contained in a single vessel (211) or being arranged in different vessels (311, 321; 411, 421; 511, 521) or compartments (522A - 522C) of vessels.

6. A process according to any of the preceding claims, further comprising a third reaction zone (S3), or stripping zone, fed with said second liquid product obtained in the second zone (S2), and where a carbamate contained in said second liquid product is decomposed by means of a heat supply and optionally by means of addition of a stripping medium, releasing ammonia and carbon dioxide, and the liquid phase in said third reaction zone being kept in a stirred condition.

7. A process according to claim 6, where a gaseous stream comprising at least part of said ammonia and carbon dioxide released in the third reaction zone is fed directly (102) in the gaseous state into said first reaction zone.

8. A reaction section of a plant for synthesis of urea from ammonia and carbon dioxide, comprising:
- a first reaction zone (S1) for conversion of ammonia and carbon dioxide into ammonium carbamate and a second reaction zone (S2) for decomposition of carbamate into urea, said second reaction zone being distinguished from said first reaction zone;
- means for feeding ammonia and carbon dioxide to said first reaction zone, and cooling means disposed in the first reaction zone and adapted to remove the heat of formation of ammonium carbamate,
- means for feeding a first product, mainly comprising ammonium carbamate, ammonia and water, from said first reaction zone to said second reaction zone;
- heating means disposed in said second reaction zone, adapted to provide heat for the decomposition of part of said carbamate into urea, and a flow line for removing a second product containing urea, residual unconverted carbamate and excess ammonia from said second reaction zone, and
- stirring means arranged in at least one of said first reaction zone and second reaction zone.

9. A reaction section according to claim 8, further comprising a third reaction zone (S3), or stripping zone; means feeding a flow of said second liquid product from the second zone (S2) to said third zone; heating means an optionally a line for addition of a stripping medium to said third zone; stirring means for keeping the liquid phase in said third reaction zone in a stirred condition.

10. A reaction section according to claim 9, further comprising a gas flow line (102, 231, 335) for a direct connection between said third zone and first zone, arranged to recycle a gaseous flow of ammonia and carbon dioxide released in the third reaction zone into said first reaction zone.

11. A reaction section according to claim 10, said gas flow line being arranged to direct said gaseous flow close to stirring means (217, 317) acting in the first reaction zone.

12. A reaction section according to any of claims 8 to 11, comprising a pressure vessel which contains both said first reaction zone and second reaction, the first reaction zone being preferably arranged above the second reaction zone in the pressure vessel.

13. A reaction section according to claim 12, said pressure vessel being an elongated vertical pressure vessel, said first zone being in a top part of the vessel and said second zone being in a mid part of the vessel.

14. A reaction section according to claim 13, said vertical pressure vessel (211) comprising:
- a top part including a cooling coil (219) and a top impeller (217) and which constitutes the first reaction zone;
- a mid part including a heating coil (229) and which constitutes the second reaction zone;
- a lower end which comprises a heating coil (239) and a bottom impeller (237) and constitutes the third reaction zone;
- said vessel being crossed by the liquid stream downwards from the top part to the mid part and the lower end, which are in a fluid communication, and
- said vessel comprising a recovery line (231) arranged for directing a gaseous stream comprising ammonia and carbon dioxide to flow upwards in the vessel from the third zone in the lower end, to the first reaction zone in the top end.

15. A reaction section according to any of claims 8 to 11, comprising:
- a first pressure vessel (311, 411, 511) containing the first reaction zone, and comprising a heat exchanger for cooling the first reaction zone, and a first impeller for providing a stirred condition of the liquid phase in said first reaction zone, and
- a second pressure vessel (321, 521) containing the second reaction zone, and comprising at least one heat exchanger for heating the second reaction zone, and at least one second impeller for providing a stirred condition of the liquid phase in said second reaction zone.

16. A reaction section according to claim 15, said second pressure vessel (521) comprising a cascade of compartments (522A, 522B, 522C), each compartment being a respective portion of said second reaction zone and having a respective heat exchanger and impeller.

17. A reaction section according to any of claims 8 to 11, comprising:
- a first pressure vessel (311, 411, 511) containing the first reaction zone, and comprising a heat exchanger for cooling the first reaction zone, and a first impeller for providing a stirred condition of the liquid phase in said first reaction zone, and
- a plurality of second pressure vessels (421A, 421 B, 421C) arranged in a cascade, each of said second vessels containing a respective portion of said second reaction zone and having a respective heat exchanger and impeller.
